# EUROPÄISCHE PATENTSCHRIFT

(11) **EP 4 132 603 B1**
(45) Veröffentlichungstag und Bekanntmachung des Hinweises auf die Patenterteilung: **28.05.2025**
(21) Anmeldenummer: 21717807.8
(22) Anmeldetag: 06.04.2021
(51) Int. Cl.: A61M 1/16, B60T 8/40, F04B 17/03, F04B 19/22, H02N 2/04

(54) **BILANZKAMMERSYSTEM FÜR EIN DIALYSEGERÄT MIT EINEM DIALYSATOR ZUR EXTRAKORPORALEN BLUTBEHANDLUNG**
BALANCE CHAMBER SYSTEM FOR A DIALYSIS MACHINE COMPRISING A DIALYSER FOR EXTRACORPOREAL BLOOD TREATMENT
SYSTÈME DE CHAMBRE D'ÉQUILIBRAGE POUR UNE MACHINE DE DIALYSE COMPRENANT UN DIALYSEUR POUR LE TRAITEMENT EXTRACORPOREL DU SANG

(30) Priorität: 08.04.2020 DE 102020204554
(43) Veröffentlichungstag der Anmeldung: 15.02.2023
(73) Patentinhaber: B. Braun Avitum AG, 34212 Melsungen (DE)
(72) Erfinder: WÜRSCHMIDT, Tobias, 34346 Hann.Münden (DE)
(74) Vertreter: Patentanwälte Ruff, Wilhelm, Beier, Dauster & Partner mbB
(86) Internationale Anmeldenummer: PCT/EP2021/058891
(87) Internationale Veröffentlichungsnummer: WO 2021/204773

(56) Entgegenhaltungen:
- DE-A1- 102014 218 981
- US-A1- 2007 164 641

## Beschreibung

Die Erfindung betrifft ein Bilanzkammersystem gemäß dem Oberbegriff des Anspruchs 1 für ein Dialysegerät.

Ein Bilanzkammersystem ist aus der DE 10 2017 125 962 A1 bekannt und wird dort als Stand der Technik gewürdigt. Das bekannte Bilanzkammersystem ist für ein Dialysegerät mit einem Dialysator zur extrakorporalen Blutbehandlung vorgesehen. Bei einer solchen Blutbehandlung wird Blut in einem extrakorporalen Blutkreislauf durch eine Blutkammer des Dialysators geleitet. Die Blutkammer ist mittels einer semipermeablen Membran von einer Dialysierflüssigkeitskammer des Dialysators getrennt, die von einer Dialysierflüssigkeit durchströmt wird. Dabei diffundieren harnpflichtige Stoffe aus dem Blut über die semipermeable Membran von der Blutkammer in die Dialysierflüssigkeitskammer, während gleichzeitig im Blut und in der Dialysierflüssigkeit vorhandene Elektrolyte von der Kammer höherer Konzentration zur Kammer niedrigerer Konzentration diffundieren. Zusätzlich kann hierbei eine sog. Ultrafiltration erwünscht sein, bei welchen dem Blut zwecks Entwässerung Flüssigkeit entzogen wird. Es ist von entscheidender Bedeutung, dass dieser Flüssigkeitsentzug bilanziert wird, denn schon ein geringfügig zu großer Flüssigkeitsentzug kann schwerwiegende medizinische Folgen haben. Das bekannte Bilanzkammersystem dient einer solchen Bilanzierung und weist eine - in einem betriebsfertig montierten Zustand - fluidleitend an den Dialysator angeschlossene Bilanzkammereinheit mit einer ersten Bilanzkammer und einer zweiten Bilanzkammer auf. Die erste Bilanzkammer ist zur Aufnahme und Abgabe von frischstromseitiger Dialysierflüssigkeit vorgesehen und an einen Zulauf des Dialysators angeschlossen. Die zweite Bilanzkammer ist zur Aufnahme und Abgabe von brauchstromseitiger Dialysierflüssigkeit vorgesehen und an einen Ablauf des Dialysators angeschlossen. Die erste Bilanzkammer und die zweite Bilanzkammer sind mittels einer zwischen unterschiedlichen Auslenkungspositionen auslenkbaren Membraneinheit fluiddicht voneinander getrennt und volumetrisch miteinander gekoppelt. In Folge der volumetrischen Kopplung verdrängt die Membraneinheit bei einer Aufnahme eines Volumens von frischstromseitiger Dialysierflüssigkeit in der ersten Bilanzkammer ein entsprechendes Volumen von brauchstromseitiger Dialysierflüssigkeit aus der zweiten Bilanzkammer und umgekehrt. Zum wechselweisen Befüllen und Entleeren der Bilanzkammern - und damit zur Förderung der Dialysierflüssigkeit - ist bei dem bekannten Bilanzkammersystem eine aufwendige Anordnung vorgesehen, die eine frischstromseitige Pumpe, eine brauchstromseitige zweite Pumpe sowie jeweils zu- und ablaufseitig der Bilanzkammern angeordnete Schaltventile aufweist.

Weiter ist aus der DE 10 2014 218 981 A1 eine physikalisch als Bilanzkammersystem fungierende Druckerzeugungseinrichtung zur Verwendung in einer Bremsanlage eines Kraftfahrzeugs bekannt. Die bekannte Druckerzeugungseinrichtung weist wenigstens eine in einer Arbeitskammer befindliche Fluiddruckkammer und einen Aktor aus einem elektroaktiven Material auf, welcher in seiner Länge und seiner Querschnittfläche veränderlich sowie in der Fluiddruckkammer positioniert ist. Der verbleibende Raum der Fluiddruckkammer abzüglich des eingefügten Aktors definiert einen Fluidraum für ein Fluid. Der Aktor bewirkt mittels einer Kombination einer Längenänderung sowie einer Änderung der Querschnittfläche eine Volumenänderung des Fluidraums. Die Volumenänderung führt zu einer Krafterzeugung auf das Fluid. Diese Kraft kann dafür verwendet werden, das Fluid anzusaugen oder aus dem Raum heraus zu pressen.

Weiter sind aus der US 2007/164641 A1 Pumpvorrichtungen mit wenigstens einem elektroaktiven Polymerwandler bekannt. Der elektroaktive Polymerwandler kann mit einem Fluid in Kontakt stehen und thermodynamische Arbeit an dem Fluid verrichten. Die bekannten Pumpvorrichtungen können insbesondere in Kühlsystemen und Heizungssystemen verwendet werden. Auch eine Verwendung in Dialysegeräten wird allgemein beschrieben.

Aufgabe der Erfindung ist es, ein Bilanzkammersystem der eingangs genannten Art bereitzustellen, das einen gegenüber dem Stand der Technik vereinfachten Aufbau aufweist.

Diese Aufgabe wird durch das Bereitstellen eines Bilanzkammersystems mit den Merkmalen des Anspruchs 1 gelöst. Durch die erfindungsgemäße Lösung kann insbesondere auf gesonderte frisch- und brauchstromseitige Pumpen zum Befüllen und Entleeren der Bilanzkammern verzichtet werden. Hierdurch kann ein vereinfachter Aufbau des Bilanzkammersystems erreicht und letztlich Aufwand bei einer Herstellung, Montage und/oder Wartung eingespart werden. Anstelle zweier solcher Pumpen ist erfindungsgemäß das wenigstens eine aus dem dielektrischen Elastomermaterial gebildete Aktorelement vorgesehen, das auch als dielektrischer Elastomer-Aktor bezeichnet werden kann. Solche Aktoren, deren grundsätzlicher Aufbau sowie das zugrundeliegende Funktionsprinzip zur Wandlung von elektrischer Energie in mechanische Energie sind jedenfalls im Bereich der Antriebstechnik grundsätzlich bekannt. Der prinzipielle Aufbau eines solchen dielektrischen Elastomer-Aktors weist einen passiven Elastomerfilm auf, dessen Ober- und Unterseite jeweils mit einer nachgiebigen Elektrode beschichtet ist. Wird eine elektrische Spannung an die einander gegenüberliegenden Elektroden angelegt, ziehen sich diese aufgrund elektrostatischer Kräfte an. Hierdurch wird der Elastomerfilm in Dickenrichtung zusammengedrückt und in seitlicher Richtung dementsprechend ausgedehnt. In einem spannungslosen Zustand kehrt der Elastomerfilm in seinem in seitlicher Richtung zusammengezogenen und in Dickenrichtung ausgedehnten Ursprungszustand zurück. Auf Grundlage dieses Funktionsprinzips lässt sich die Stellbewegung zur Auslenkung der Membraneinheit generieren. Der Elastomerfilm kann insbesondere aus Silikon oder Acryl gefertigt sein. Die Elektroden können insbesondere aus Graphitpulver, Silikonöl-Graphitgemisch oder Gold gefertigt sein. Ausgehend von dem vorbeschriebenen grundsätzlichen Aufbau kann das wenigstens eine Aktorelement insbesondere in Form eines Planaraktors, Stapelaktors, Bündelaktors, Rollaktors oder Schalenaktors ausgebildet sein. Die vorgenannten Bauformen sind als solches grundsätzlich bekannt, sodass von weitergehenden diesbezüglichen Erläuterungen abgesehen wird. Das wenigstens eine Aktorelement kann insbesondere in die Membraneinheit integriert sein oder abseits der Membraneinheit angeordnet und mechanisch mit derselben gekoppelt sein. Es versteht sich, dass die erste Bilanzkammer und die zweite Bilanzkammer jeweils einen Zulauf und einen fluidleitend mit dem Zulauf verbundenen Ablauf aufweisen. Der Zulauf der ersten Bilanzkammer ist in betriebsfertig montierten Zustand an eine Quelle der Dialysierflüssigkeit angeschlossen. Der Ablauf der ersten Bilanzkammer ist zum Anschluss an einen Zulauf einer Dialysierflüssigkeitskammer des Dialysators vorgesehen. Der Zulauf der zweiten Bilanzkammer ist zum Anschluss an einen Ablauf der Dialysierflüssigkeitskammer des Dialysators vorgesehen. Der Ablauf der zweiten Bilanzkammer ist in betriebsfertig montierten Zustand an einen Abfluss zur Entsorgung und/oder Aufbereitung der Dialysierflüssigkeit angeschlossen. Vorzugsweise kann das Bilanzkammersystem eine zweite Bilanzkammereinheit aufweisen, die sowohl frischals auch brauchstromseitig fluidleitend parallel zu der ersten Bilanzkammereinheit angeordnet ist. Hierdurch kann eine kontinuierliche Förderung der Dialysierflüssigkeit durch den Dialysator erreicht werden. Die Dialysierflüssigkeitskammer umfasst diejenigen fluidführenden Abschnitte des Dialysators, die von Dialysierflüssigkeit durchflossen werden. Diejenigen fluidführenden Abschnitte des Dialysators, die von Blut durchflossen werden, können als Blutkammer bezeichnet werden. Die Dialysierflüssigkeit kann auch als Dialysat bezeichnet werden.

In Ausgestaltung der Erfindung ist das wenigstens eine Aktorelement zur kapazitiven Erfassung der Auslenkungsposition der Membraneinheit eingerichtet und fungiert somit zusätzlich als Sensorelement. Die vorbeschriebene Geometrieänderung des wenigstens einen Aktorelements unter Einwirkung der elektrischen Spannung bewirkt gleichzeitig eine Kapazitätsänderung, die für sensorische Zwecke, beispielsweise zur Druck- und/oder Wegmessung genutzt werden kann. Dem wenigstens einen Aktorelement kommt demnach bei dieser Ausgestaltung der Erfindung eine besonders vorteilhafte Mehrfachfunktion zu. Infolge dessen kann insbesondere auf eine gesonderte Sensorik zur Erfassung der Auslenkungsposition der Membraneinheit verzichtet werden, wodurch ein nochmals vereinfachter Aufbau des Bilanzkammersystems erreicht werden kann.

In weiterer Ausgestaltung der Erfindung ist wenigstens ein Federelement mit der Membraneinheit und dem Aktorelement wirkverbunden, wobei die Membraneinheit - bei an dem Aktorelement angelegter elektrischer Spannung - mittels des Federelements in eine erste Richtung ausgelenkt ist, und wobei die Membraneinheit - bei verringerter elektrischer Spannung - mittels des Aktorelements entgegen der Einwirkung des Federelements in eine der ersten Richtung entgegengesetzte zweite Richtung ausgelenkt ist. Durch diese Ausgestaltung der Erfindung wird mit konstruktiv einfachen Mitteln eine Auslenkung der Membraneinheit in zwei Richtungen, nämlich die erste Richtung und die zweite Richtung, erreicht. Zu diesem Zweck ist das Federelement vorgesehen und gleichsam in einer Parallelschaltung zu dem Aktorelement mit der Membraneinheit wirkverbunden. Das Federelement kann insbesondere als Wendel- oder Blattfeder gestaltet und aus Metall oder Kunststoff gefertigt sein. Das Federelement kann in die Membraneinheit integriert sein. Alternativ kann das Federelement abseits der Membraneinheit angeordnet und mechanisch mit der Membraneinheit wirkverbunden sein. Vorzugsweise ist das wenigstens eine Federelement an einem Lagerabschnitt der Bilanzkammereinheit abgestützt, der der ersten Bilanzkammer und/oder der zweiten Bilanzkammer zugeordnet sein kann.

Weiter gemäß der Erfindung weist die Membraneinheit eine die erste Bilanzkammer begrenzende erste Membran und eine die zweite Bilanzkammer begrenzende zweite Membran auf, wobei die erste Membran und die zweite Membran zur volumetrischen Kopplung der ersten Bilanzkammer und der zweiten Bilanzkammer mittels eines starren Koppelelements und/oder einer inkompressiblen Koppelflüssigkeit miteinander wirkverbunden sind. Eine solche Ausgestaltung kann insbesondere dann vorteilhaft sein, wenn die erste Bilanzkammer und die zweite Bilanzkammer bauraumbedingt nicht unmittelbar aneinander angrenzend in einem geräteseitig zur Verfügung stehenden Bauraum angeordnet werden können. Um dennoch die zur Bilanzierung notwendige volumetrische Kopplung zwischen der ersten Bilanzkammer und der zweiten Bilanzkammer zu gewährleisten, sind das Koppelelement und/oder die Koppelflüssigkeit vorgesehen. Das Koppelelement und/oder die Koppelflüssigkeit bilden eine Wirkverbindung zwischen der ersten und der zweiten Membran. Das starre Koppelelement ist einends an der ersten Membran und andernends an der zweiten Membran abgestützt, sodass eine Auslenkung der ersten Membran mittels des Koppelelements auf die zweite Membran übertragen wird und umgekehrt. Die Koppelflüssigkeit kann in einen zwischen der ersten und der zweiten Membran angeordneten Aufnahmeraum eingefüllt sein. Um eine volumengleiche Auslenkung beider Membranen zu gewährleisten, ist die Koppelflüssigkeit inkompressibel und beispielsweise Wasser, Öl, Silikon oder ein aus wenigstens zwei der vorgenannten Flüssigkeiten gebildetes Gemisch. Das wenigstens eine Aktorelement kann in die erste Membran oder die zweite Membran integriert sein. Alternativ können zwei Aktorelemente vorgesehen sein, wobei in jede der Membranen eines der Aktorelemente integriert ist. Weiter alternativ kann das wenigstens eine Aktorelement abseits beider Membranen angeordnet und mit wenigstens einer der Membranen mechanisch wirkverbunden sein. Alternativ kann eine mechanische Wirkverbindung mit beiden Membranen vorgesehen sein. Sofern ein zu dem wenigstens einen Aktorelement parallel geschaltetes Federelement vorgesehen ist, gilt im Hinblick auf eine Integration oder abseitige Anordnung desselben das zu dem wenigstens einen Aktorelement Gesagte in entsprechender Weise.

In weiterer Ausgestaltung der Erfindung ist ein einziges Aktorelement vorgesehen, dessen Stellbewegung mittels des Koppelelements und/oder der Koppelflüssigkeit zwischen der ersten Membran und der zweiten Membran übertragen ist. Diese Ausgestaltung der Erfindung ermöglicht die Auslenkung beider Membranen mittels lediglich eines Aktorelements, sodass ein insoweit einfacher Aufbau erreicht ist.

In weiterer Ausgestaltung der Erfindung ist das wenigstens eine Aktorelement in Gestalt wenigstens einer aktiven Membranschicht in die Membraneinheit, insbesondere in die erste Membran und/oder die zweite Membran, integriert. Dies ist eine besonders vorteilhafte Ausgestaltung der Erfindung, da insbesondere im Vergleich zu einer Anordnung des wenigstens einen Aktorelements abseits der Membraneinheit Bauraum eingespart werden kann. Das wenigstens eine Aktorelement liegt bei dieser Ausgestaltung der Erfindung demnach vorzugsweise als Planar- oder Stapelaktor vor. In erstgenanntem Fall ist eine einzige ober- und unterseitig mit Elektroden beschichtete Elastomerschicht vorgesehen. In zweitgenanntem Fall sind mehrere Planaraktoren übereinandergestapelt, wodurch insbesondere vergleichsweise größere Stellkräfte und/oder Stellbewegungen erreicht werden können.

In weiterer Ausgestaltung der Erfindung ist die aktive Membranschicht mittels wenigstens einer weiteren Membranschicht und/oder einer Beschichtung gegenüber einer Benetzung mit der Dialysierflüssigkeit geschützt. Durch diese Ausgestaltung der Erfindung werden unerwünschte chemische und/oder elektrochemische Reaktionen zwischen der aktiven Membranschicht und der Dialysierflüssigkeit vermieden. Die weitere Membranschicht und/oder Beschichtung kann insbesondere aus Silikon gefertigt sein. Vorzugsweise ist die aktive Membranschicht zwischen zwei weiteren Membranschichten angeordnet oder beidseits beschichtet. Vorzugsweise ist das wenigstens eine Aktorelement bzw. die aktive Membranschicht unter Ausbildung der Membraneinheit oder einer der Membranen der Membraneinheit mit Silikon umgossen.

In weiterer Ausgestaltung der Erfindung ist wenigstens eine aus einem dielektrischen Elastomermaterial gebildete Sensorschicht in die Membraneinheit integriert, wobei die wenigstens eine Sensorschicht zur Erfassung eines in der ersten Bilanzkammer und/oder der zweiten Bilanzkammer vorherrschenden Drucks eingerichtet ist. Dies ist eine besonders bevorzugte Ausgestaltung der Erfindung. Denn durch die wenigstens eine Sensorschicht kann auf eine von der Membraneinheit getrennte Sensorik zur Druckerfassung in der ersten Bilanzkammer und/oder der zweiten Bilanzkammer verzichtet werden. Stattdessen ist für eine solche Druckerfassung die wenigstens eine Sensorschicht vorgesehen und in die Membraneinheit integriert. Bei einer Erhöhung des Drucks ändert sich die Kapazität der wenigstens einen Sensorschicht. Dies geschieht aufgrund der dielektrischen Eigenschaften des Elastomermaterials der wenigstens einen Sensorschicht. In Abhängigkeit der Änderung der Kapazität kann der Druck ermittelt werden.

In weiterer Ausgestaltung der Erfindung ist das wenigstens eine Aktorelement in Gestalt eines Linearaktors mit einer translatorischen Stellbewegung abseits der Membraneinheit angeordnet. Dementsprechend ist das wenigstens eine Aktorelement bei dieser Ausgestaltung der Erfindung nicht in die Membraneinheit integriert. Stattdessen wirkt das wenigstens eine Aktorelement quasi von außen auf die Membraneinheit. Weiter ist hierbei eine Gestaltung des wenigstens einen Aktorelements als Linearaktor vorgesehen, der zur Ausführung einer translatorischen Stellbewegung eingerichtet ist. Hierfür kann der Linearaktor insbesondere ein aus mehreren Stapelaktoren gebündelter Bündelaktor sein.

In weiterer Ausgestaltung der Erfindung weisen die Bilanzkammern jeweils einen Zulauf und einen Ablauf auf, wobei zur Flusssteuerung durch den jeweiligen Zu- oder Ablauf jeweils ein Rückschlagventil vorgesehen ist. Dies ist eine besonders bevorzugte Ausgestaltung der Erfindung. Demgegenüber sind im eingangs genannten Stand der Technik zur Flusssteuerung Schaltventile notwendig. Durch die erfindungsgemäße Lösung kann auf solche Schaltventile zur Flusssteuerung verzichtet werden. Stattdessen sind vergleichsweise einfach aufgebaute, kostengünstige Rückschlagventile ausreichend. Die Rückschlagventile können insbesondere jeweils als Tellerventil gestaltet sein.

Die Erfindung betrifft zudem ein Dialysegerät mit einem Dialysator zur extrakorporalen Blutbehandlung, wobei ein Bilanzkammersystem nach einem der vorhergehenden Ansprüche fluidleitend mit einer Dialysierflüssigkeitskammer des Dialysators verbunden ist.

Im Übrigen kann ein aus dem dielektrischen Elastomermaterial gebildetes Aktorelement einer Schlauchklemme und/oder einem Absperrventil des Dialysegeräts zugeordnet sein. Das Aktorelement kann in diesem Fall unter Einwirkung der elektrischen Spannung eine Stellbewegung zum Betätigen der Schlauchklemme oder des Absperrventils bewirken.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt eine schematische Darstellung eines Abschnitts eines aus dem Stand der Technik bekannten Dialysegeräts, das mit einem aus dem Stand der Technik bekannten Bilanzkammersystem versehen ist,
- Fig. 2: eine schematische Darstellung eines Abschnitts einer beispielhaften Ausführungsform eines vereinfachten Dialysegeräts, das mit einer Ausführungsform eines Bilanzkammersystems nach dem Stand der Technik gemäß DE 10 2014 218 981 A1 (siehe oben) versehen ist,
- Fig. 3: in schematisch stark vereinfachter beispielhafter Darstellung eine Bilanzkammereinheit des in Fig. 2 gezeigten Bilanzkammersystems,
- Fig. 4: in schematisch stark vereinfachter beispielhafter Querschnittsdarstellung einen Abschnitt einer Membraneinheit des Bilanzkammersystems nach Fig. 3 in einem Bereich IV,
- Fig. 5: in einer der Fig. 3 entsprechenden Darstellungsweise eine erfindungsgemäße Ausgestaltung einer Bilanzkammereinheit für das Bilanzkammersystem nach Fig. 2,
- Fig. 6: in einer der Fig. 3 entsprechenden Darstellungsweise eine weitere erfindungsgemäße Ausgestaltung einer Bilanzkammereinheit für das Bilanzkammersystem nach Fig. 2,
- Fig. 7: in einer der Fig. 3 entsprechenden Darstellungsweise eine weitere beispielhafte, nicht erfindungsgemäße Ausgestaltung einer Bilanzkammereinheit für das Bilanzkammersystem nach Fig. 2,
- Fig. 8: in einer der Fig. 3 entsprechenden Darstellungsweise eine weitere erfindungsgemäße Ausgestaltung einer Bilanzkammereinheit für das Bilanzkammersystem nach Fig. 2,
- Fig. 9: in einer der Fig. 3 entsprechenden Darstellungsweise eine weitere erfindungsgemäße Ausgestaltung einer Bilanzkammereinheit für das Bilanzkammersystem nach Fig. 2,
- Fig. 10: in einer der Fig. 3 entsprechenden Darstellungsweise eine weitere erfindungsgemäße Ausgestaltung einer Bilanzkammereinheit für das Bilanzkammersystem nach Fig. 2,
- Fig. 11: in schematisch stark vereinfachter Perspektivdarstellung eine weitere Ausgestaltung einer erfindungsgemäßen Bilanzkammereinheit für das Bilanzkammersystem nach Fig. 2 in Blickrichtung auf eine Membraneinheit und
- Fig. 12: eine schematisch vereinfachte Darstellung eines Schichtaufbaus der Membraneinheit nach Fig. 11.

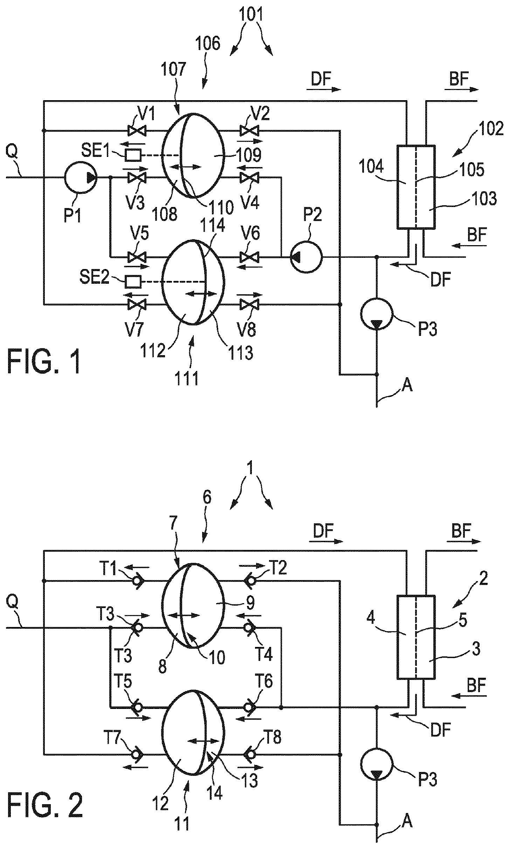

Fig. 1 zeigt in schematisch stark vereinfachter Darstellung einen Ausschnitt eines aus dem Stand der Technik bekannten Dialysegeräts 101, das zur extrakorporalen Blutbehandlung vorgesehen ist und einen Dialysator 102 mit einer Blutkammer 103 und einer Dialysierflüssigkeitskammer 104 aufweist. Die Blutkammer 103 ist mittels einer semipermeablen Membran 105 von der Dialysierflüssigkeitskammer 104 getrennt und fluidleitend an einen nicht näher bezeichneten extrakorporalen Blutkreislauf mit einer in Fig. 1 angedeuteten Fließrichtung BF des zu behandelnden Blutes angeschlossen. Weiter weist das bekannte Dialysegerät 101 ein aus dem Stand der Technik bekanntes Bilanzkammersystem 106 auf, das fluidleitend an die Dialysierflüssigkeitskammer 104 angeschlossen ist.

Das bekannte Bilanzkammersystem 106 dient in einer dem Fachmann bekannten Weise einer volumetrischen Bilanzierung einer durch den Dialysator 102 geleiteten Dialysierflüssigkeit, die im Betrieb des Dialysegeräts 101 entlang einer anhand Fig. 1 angedeuteten Fließrichtung DF gefördert wird.

Das bekannte Bilanzkammersystem 106 weist eine erste Bilanzkammereinheit 107 mit einer ersten Bilanzkammer 108 und einer zweiten Bilanzkammer 109 auf. Die erste Bilanzkammer 108 und die zweite Bilanzkammer 109 sind mittels einer zwischen unterschiedlichen Auslenkungspositionen auslenkbaren Membraneinheit 110 fluiddicht voneinander getrennt und volumetrisch miteinander gekoppelt. Die erste Bilanzkammer 108 ist zur Aufnahme und Abgabe von frischstromseitiger Dialysierflüssigkeit vorgesehen und kann daher auch als frischstromseitige erste Bilanzkammer 108 bezeichnet werden. Die zweite Bilanzkammer 109 ist demgegenüber zur Aufnahme und Abgabe von brauchstromseitiger Dialysierflüssigkeit vorgesehen und kann daher auch als brauchstromseitige zweite Bilanzkammer 109 bezeichnet werden. Zum Befüllen der ersten Bilanzkammer 108 und der zweiten Bilanzkammer 109 ist jeweils eine Pumpe, nämlich eine frischstromseitige erste Pumpe P1 und eine brauchstromseitige zweite Pumpe P2, vorgesehen. Weiter weist das bekannte Bilanzkammersystem 106 Ventile V1, V2, V3, V4 zur Flusssteuerung der Dialysierflüssigkeit durch die erste Bilanzkammer 108 und die zweite Bilanzkammer 109 auf, die zu- bzw. ablaufseitig an die Bilanzkammern 108, 109 angeschlossen und als Schaltventile gestaltet sind. Zur Verstetigung der Förderung der Dialysierflüssigkeit weist das bekannte Bilanzkammersystem eine zweite Bilanzkammereinheit 111 mit einer dritten Bilanzkammer 112 und einer vierten Bilanzkammer 113 auf, die mittels einer weiteren Membraneinheit 114 fluiddicht voneinander getrennt und volumetrisch miteinander gekoppelt sind. Die dritte Bilanzkammer 112 ist frischstromseitig parallel zu der ersten Bilanzkammer 108 geschaltet und mittels der ersten Pumpe P1 befüllbar. Die vierte Bilanzkammer 113 ist brauchstromseitig zu der zweiten Bilanzkammer 109 parallel geschaltet und mittels der zweiten Pumpe P2 befüllbar. Zur Flusssteuerung sind in einer zu der ersten Bilanzkammereinheit 107 entsprechenden Weise Ventile V5, V6, V7, V8 vorgesehen.

Die Funktion des Bilanzkammersystems 106 ist in zwei Phasen unterteilt.

In einer ersten Phase liegen die folgenden Startbedingungen vor: Erste Bilanzkammer 108 leer, zweite Bilanzkammer 109 voll, die Membraneinheit 110 nimmt eine - in Bezug auf die Zeichenebene der Fig. 1 - nach links ausgelenkte Auslenkungsposition ein, dritte Bilanzkammer 112 voll, vierte Bilanzkammer 113 leer, die Membraneinheit 114 nimmt eine nach rechts ausgelenkte Auslenkungsposition ein. Die Ventile sind wie folgt geschaltet: V1 geschlossen, V2 offen, V3 offen, V4 geschlossen, V5 geschlossen, V6 offen, V7 offen, V8 geschlossen. Die erste Pumpe P1 füllt somit die erste Bilanzkammer 108 durch das Ventil V3 mit frischer Dialysierflüssigkeit, die hierzu beispielsweise aus einer Quelle Q entnommen werden kann. Dadurch wird verbrauchte Dialysierflüssigkeit, die sich in der zweiten Bilanzkammer 109 befindet und auch als Dialysat bezeichnet werden kann, durch das Ventil V2 in einen entsprechenden Ausguss A abgeleitet. Gleichzeitig füllt die zweite Pumpe P2 die vierte Bilanzkammer 113 durch das Ventil V6 mit verbrauchter Dialysierflüssigkeit. Dadurch wird frische Dialysierflüssigkeit, die sich in der dritten Bilanzkammer 112 befindet, durch das Ventil V7 zum Dialysator 102 geleitet.

In einer zweiten Phase liegen die folgenden Startbedingungen vor: Zweite Bilanzkammer 109 leer, erste Bilanzkammer 108 voll, die Membraneinheit 110 nimmt eine nach rechts ausgelenkte Auslenkungsposition ein, vierte Bilanzkammer 113 voll, dritte Bilanzkammer 112 leer, die Membraneinheit 114 nimmt eine nach links ausgelenkte Auslenkungsposition ein. Die Ventile sind wie folgt geschaltet: V1 offen, V2 geschlossen, V3 geschlossen, V4 offen, V5 offen, V6 geschlossen, V7 geschlossen, V8 offen. Die erste Pumpe P1 füllt somit die dritte Bilanzkammer 112 durch das Ventil V5 mit frischer Dialysierflüssigkeit. Dadurch wird in der vierten Bilanzkammer 113 befindliche verbrauchte Dialysierflüssigkeit durch das Ventil V8 in den Ausguss A geleitet. Gleichzeitig füllt die zweite Pumpe P1 die zweite Bilanzkammer 109 durch das Ventil V4 mit verbrauchter Dialysierflüssigkeit. Hierdurch wird in der ersten Bilanzkammer 108 befindliche frische Dialysierflüssigkeit durch das Ventil V1 zum Dialysator 102 gefördert.

Die wechselweise Schaltung der Ventile erfolgt insbesondere in Abhängigkeit der jeweiligen Auslenkungsposition der Membraneinheiten 110, 114. Zu deren messtechnischer Erfassung sieht die gezeigte aus dem Stand der Technik bekannte Ausführungsform Sensoreinheiten SE1, SE2 vor, die jeweils als induktive Membransensoren gestaltet und einer der Bilanzkammereinheiten 107, 108 zugeordnet sind.

Im Übrigen weist das bekannte Bilanzkammersystem 106 eine brauchstromseitig angeordnete Ultrafiltrationspumpe P3 auf, die einen hydraulischen Kurzschluss zwischen den Bilanzkammereinheiten 107, 111 bildet und einem definierten Entzug von Dialysierflüssigkeit aus dem als solchen geschlossenen bilanzkammerseitigen Kreislauf dient.

Der vorbeschriebene Aufbau des bekannten Bilanzkammersystems 106 ist komplex und weist insbesondere hinsichtlich seiner Montage, Wartung und/oder Instandsetzung Nachteile auf. Dies insbesondere aufgrund der Pumpen P1, P2, der einzeln anzusteuernden Ventile V1 bis V8 und der gesonderten Sensoreinheiten SE1, SE2.

Fig. 2 zeigt in schematisch stark vereinfachter Darstellungsweise einen Ausschnitt einer beispielhaften Ausführungsform eines Dialysegeräts 1, das eine vom Aufbau her aus dem oben genannten Stand der Technik nach DE 10 2014 218 981 A1 bekannten Ausführungsform eines

Bilanzkammersystems 6 aufweist. Das Dialysegerät 1 weist in einer zu dem bekannten Dialysegerät 101 entsprechenden Weise einen Dialysator 2 mit einer Blutkammer 3, einer Dialysierflüssigkeitskammer 4, und einer semipermeablen Membran 5 auf. Zur Vermeidung von Wiederholungen wird auf die diesbezügliche Beschreibung im Zusammenhang mit Fig. 1 verwiesen, die hinsichtlich Fig. 2 in entsprechender Weise gilt.

Das Bilanzkammersystem 6 weist eine Bilanzkammereinheit 7 mit einer ersten Bilanzkammer 8 und einer zweiten Bilanzkammer 9 auf, die mittels einer zwischen unterschiedlichen Auslenkungspositionen auslenkbaren Membraneinheit 10 fluiddicht voneinander getrennt und volumetrisch miteinander gekoppelt sind. Die erste Bilanzkammer 8 ist frischstromseitig angeordnet und somit zur Aufnahme und Abgabe von frischstromseitiger Dialysierflüssigkeit vorgesehen. Die zweite Bilanzkammer 9 ist demgegenüber brauchstromseitig angeordnet und somit zur Aufnahme und Abgabe von brauchstromseitiger Dialysierflüssigkeit vorgesehen.

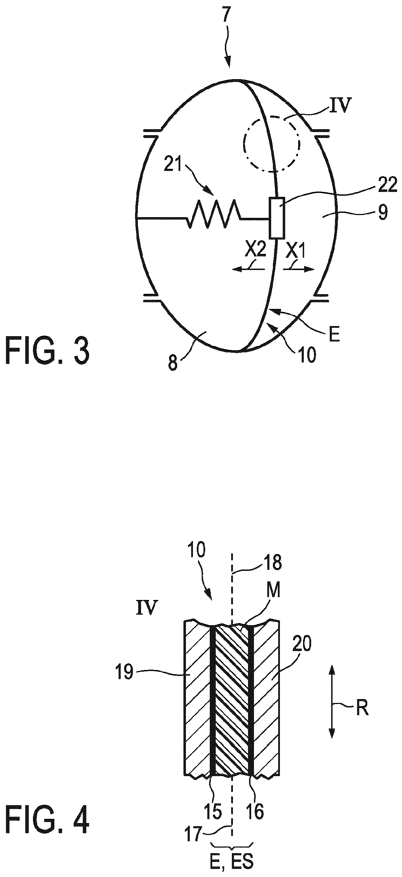

Zur Verstetigung der Förderung der Dialysierflüssigkeit durch die Dialysierflüssigkeitskammer 4 weist das Bilanzkammersystem 6 - in einer dem bekannten Bilanzkammersystem 106 grundsätzlich entsprechenden Weise - eine zweite Bilanzkammereinheit 11 auf, die bei der gezeigten Ausführungsform baugleich zu der Bilanzkammer 7 ausgeführt ist. Insoweit kann die Bilanzkammereinheit 7 vorliegend auch als erste Bilanzkammereinheit bezeichnet werden. Eine solche Ausgestaltung mit einer ersten Bilanzkammereinheit 7 und einer zweiten Bilanzkammereinheit 11 ist jedoch nicht zwingend. Eine nicht gezeigte Ausführungsform des Bilanzkammersystems weist dementsprechend lediglich eine einzige Bilanzkammereinheit auf. Bevor der weitere Aufbau und die Funktionsweise des Bilanzkammersystems 6 im Detail erläutert werden, wird insbesondere anhand der Fig. 3 bis 12 auf die Gestaltung der Bilanzkammereinheit 7 eingegangen.

Anhand der beispielhaften Darstellungen in den Fig. 3 und 4 ist gezeigt, dass ein Aktorelement E zur Auslenkung der Membraneinheit 10 und damit zur Förderung der Dialysierflüssigkeit vorgesehen ist. Das Aktorelement E ist aus einem dielektrischen Elastomermaterial M gebildet (Fig. 4). Dielektrische Elastomermaterialien zur Wandlung von elektrischer Energie in mechanischer Arbeit und die dem zugrunde liegenden Wirkprinzipien sind als solche bekannt. Dabei ist das Aktorelement E bei der Ausführungsform nach den Fig. 3 und 4 in die Membraneinheit 10 integriert und bildet auf diese Weise gleichsam eine aktive Membranschicht ES der Membraneinheit 10 (Fig. 4).

Unter Einwirkung einer elektrischen Spannung führt das Aktorelement E, das auch als dielektrischer Elastomeraktor bezeichnet werden kann, eine anhand Fig. 4 verdeutlichte Stellbewegung R aus. Beim Anlegen einer elektrischen Spannung wird das Elastomermaterial M in Dickenrichtung der Membraneinheit 10 komprimiert und hierdurch in seiner Fläche ausgedehnt; bei einem Verringern dieser Spannung und/oder in einem spannungslosen Zustand kehrt das Elastomermaterial M elastisch in seine Ursprungsstellung zurück, sodass eine Stellbewegung R entlang der in Fig. 4 eingezeichneten Richtung möglich ist. Hierdurch ist eine Auslenkung der Membraneinheit 10 entlang der in Fig. 3 eingezeichneten Richtungspfeile möglich. Die besagte elektrische Spannung kann über vorder- und rückseitig auf das dielektrische Elastomermaterial M aufgebrachte Elektroden 15, 16 angelegt werden, die auf grundsätzlich bekannte Weise elektrisch leitend an Spannungsleitungen 17, 18 eines im Übrigen nicht näher gezeigten Schalt- und/oder Steuerkreises angeschlossen sein können.

Um eine unmittelbare Kontaktierung der aktiven Membranschicht ES mit der Dialysierflüssigkeit zu vermeiden, weist die Membraneinheit 10 bei der gezeigten Ausführungsform weitere Membranschichten, nämlich eine erste Membranschicht 19 und eine zweite Membranschicht 20 auf. Die aktive Membranschicht ES ist in Dickenrichtung der Membraneinheit 10 zwischen der ersten Membranschicht 19 und der zweiten Membranschicht 20 angeordnet. Dabei versteht sich, dass die anhand Fig. 4 gezeigten Abmessungen in Dickenrichtung der Membraneinheit 10 rein exemplarisch und nicht etwa maßstäblich aufzufassen sind.

Die beispielhafte Ausführungsform nach den Fig. 3 und 4 weist zudem ein Federelement 21 auf, das mit der Membraneinheit 10 und damit auch mit dem in dieselbe integrierten Aktorelement E wirkverbunden ist. Zu diesem Zweck ist das Federelement 21 einends an einem nicht näher bezeichneten Gehäuse der Bilanzkammereinheit 7 abgestützt. Andernends ist das Federelement 21 an der Membraneinheit 10 abgestützt. Vorliegend wirkt das Federelement 21 auf eine Ankerplatte 22 der Membraneinheit 10, die auf grundsätzlich bekannte Weise fest mit den Membranschichten 19, 20, ES verbunden und vergleichsweise starr ausgeführt ist. Das Federelement 21 ist bei der gezeigten Ausführungsform als Wendelfeder gestaltet. Bei einer nicht gezeigten Ausführungsform ist das Federelement stattdessen als Blattfeder gestaltet. Dabei ist die Blattfeder in die Membraneinheit integriert.

In dem anhand Fig. 3 gezeigten Zustand ist die Membraneinheit 10 in etwa mittig zwischen der ersten Bilanzkammer 8 und der zweiten Bilanzkammer 9 angeordnet und lediglich geringfügig nach rechts ausgelenkt. Zur Auslenkung der Membraneinheit 10 und damit zur Förderung der Dialysierflüssigkeit wird das Aktorelement E, genauer: die aktive Membranschicht ES, über die Spannungsleitungen 17, 18 mit Spannung beaufschlagt und damit zum Ausführen der Stellbewegung R angesteuert, wofür eine entsprechend eingerichtete Steuereinheit vorgesehen sein kann. Eine spannungsbedingte Ausdehnung der aktiven Membranschicht ES bewirkt eine Auslenkung der Membraneinheit 10 entlang einer Richtung X1 unter Einwirkung des Federelements 21. Ein Verringern der angelegten Spannung bewirkt eine Kontraktion der aktiven Membranschicht ES, wodurch die Membraneinheit 10 entlang einer Richtung X2 entgegen der Einwirkung des Federelements 21 ausgelenkt wird.

Gegenüber dem aus dem Stand der Technik gemäß DE 10 2017 125 962 A1 (siehe eingangs oben) bekannten Bilanzkammersystem 106 kann hierdurch auf die dortigen Pumpen P1, P2 zum frischstrom- und brauchstromseitigen Befüllen der Bilanzkammereinheit 7 und damit zur Förderung der Dialysierflüssigkeit verzichtet werden. Vielmehr erfolgt die Förderung mittels des Aktorelements E, das bei der beispielhaften Ausführungsform nach den Fig. 3 und 4 in die Membraneinheit 10 integriert ist. Dies führt zu einer maßgeblichen Vereinfachung des konstruktiven Aufbaus.

Bei der beispielhaften Ausführungsform nach den Fig. 3 und 4 ist das Aktorelement E zudem zur kapazitiven Erfassung der Auslenkungsposition der Membraneinheit 10 eingerichtet. Somit fungiert das Aktorelement E zusätzlich als Sensorelement. Hierdurch kann im Vergleich zu dem aus dem

Stand der Technik gemäß DE 10 2017 125 962 A1 bekannten Bilanzkammersystem 106 auf die dort üblichen, gesonderten induktiven Membransensoren SE1, SE2 verzichtet und ein weiter vereinfachter Aufbau des Bilanzkammersystems 6 erreicht werden. Die Änderung der Kapazität des Aktorelements E resultiert aus dessen Geometrieänderung bei der Auslenkung der Membraneinheit 10 entlang der Richtungen X1, X2, wobei die dem zugrunde liegenden physikalischen Effekte als solches bekannt sind. Die mittels des Aktorelements E erfasste Auslenkungsposition der Membraneinheit 10 kann, beispielsweise mittels vorgenannter Steuereinheit, steuerungs- und/oder regelungstechnisch verwertet werden.

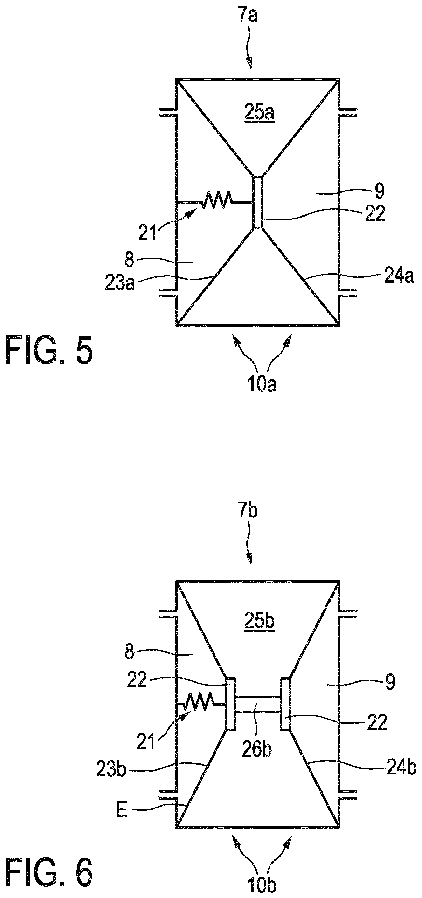

Anhand der Fig. 5, 6 und 8 bis 12 sind jeweils unterschiedlich ausgeführte erfindungsgemäße Bilanzkammereinheiten 7a, 7b und 7d bis

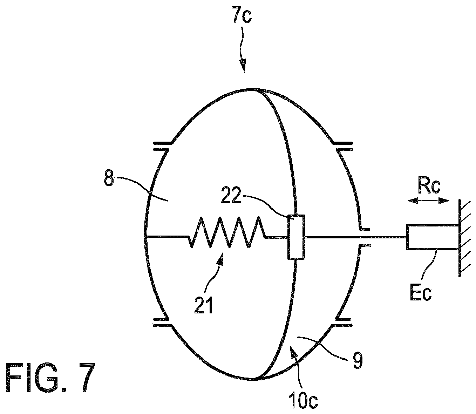

7g gezeigt. Fig. 7 zeigt eine weitere beispielhafte, nicht erfindungsgemäße Ausführung einer Bilanzkammereinheit 7c. Zur Vermeidung von Wiederholungen wird nachfolgend lediglich auf die wesentlichen Unterschiede der Bilanzkammereinheiten 7a bis 7g gegenüber der Bilanzkammereinheit 7 nach Fig. 3 eingegangen. Identische Bauteile und/oder Abschnitte sind insoweit mit identischen Bezugszeichen versehen und werden nicht gesondert erläutert. Stattdessen wird auf die Ausführungsform nach den Fig. 3 und 4 verwiesen. Unterschiedlich ausgeführte Bauteile und/oder Abschnitte sind mit gleichen Bezugszeichenziffern unter Hinzufügung von entsprechenden Kleinbuchstaben bezeichnet.

Die Bilanzkammereinheit 7a nach Fig. 5 unterscheidet sich im Wesentlichen dadurch von der Bilanzkammereinheit 7, dass die Membraneinheit 10a eine erste Membran 23a und eine zweite Membran 24a aufweist. Demgegenüber weist die Membraneinheit 10 nach den Fig. 3 und 4 lediglich eine einzige nicht näher bezeichnete Membran auf, die aus den anhand Fig. 4 ersichtlichen Membranschichten gebildet ist und deren Vorderseite die zweite Bilanzkammer 9 begrenzt und deren gegenüberliegende Rückseite die erste Bilanzkammer 8 begrenzt.

Stattdessen ist bei der Bilanzkammereinheit 7a nach Fig. 5 vorgesehen, dass die erste Membran 23a, die erste Bilanzkammer 8 begrenzt; die zweite Membran 24a begrenzt die zweite Bilanzkammer 9. Zur volumetrischen Kopplung zwischen der ersten Bilanzkammer 8 und der zweiten Bilanzkammer 9 ist eine inkompressible Koppelflüssigkeit 25a fluiddicht zwischen einander gegenüberliegenden Flächen der ersten Membran 23a und der zweiten Membran 24a eingeschlossen. Die inkompressible Koppelflüssigkeit 25a gewährleistet, dass die zweite Membran 24a bei einer Auslenkung der ersten Membran 23a entsprechend volumengleich ausgelenkt wird und umgekehrt.

Sowohl die erste Membran 23a als auch die zweite Membran 24a greifen vorliegend an der Ankerplatte 22 der Membraneinheit 10a an, was jedoch nicht zwingend notwendig ist. Bei einer nicht gezeigten Ausführungsform können dementsprechend gesonderte Ankerplatten vorgesehen und jeweils lediglich einer der Membranen zugeordnet sein.

Zur Auslenkung der Membraneinheit 10a und damit zur Förderung der Dialysierflüssigkeit weist wenigstens eine der Membranen 23a, 24a den bereits anhand der Fig. 3 und 4 erläuterten Aufbau mit einer aktiven Membranschicht ES auf. Vorliegend ist vorgesehen, dass die erste Membran 23a eine aktive Membranschicht ES (vgl. Fig. 4) aufweist. Alternativ können beide Membranen 23a, 24a mit einer aktiven Membranschicht ES versehen sein.

Die Bilanzkammereinheit 7b nach Fig. 6 unterscheidet sich im Wesentlichen dadurch von der Bilanzkammereinheit 7a nach Fig. 5, dass zur volumetrischen Kopplung zwischen der ersten Membran 23b und der zweiten Membran 24b ein starres Koppelelement 26b vorgesehen ist. Das Koppelelement 26b ist einends an der ersten Membran 23b und andernends an der zweiten Membran 24b abgestützt und dient einer Übertragung der jeweiligen Auslenkung zwischen den Membranen 23b, 24b. Zusätzlich ist eine inkompressible Koppelflüssigkeit 25b fluiddicht zwischen einander gegenüberliegenden Flächen der ersten Membran 23b und der zweiten Membran 24b eingeschlossen. Bei einer nicht gezeigten Ausführungsform ist hierauf verzichtet, sodass einzig das Koppelelement 26b zur Bewegungsübertragung zwischen den Membranen 23b, 24b vorgesehen ist. Bei der Bilanzkammereinheit 7b nach Fig. 6 weist lediglich die erste Membran 23b eine aktive Membranschicht ES (vgl. Fig. 4) auf. Auch der übrige Aufbau der ersten Membran 23b entspricht der anhand von Fig. 4 erläuterten Gestaltung. Demgegenüber ist die zweite Membran 24b quasi als "passive" Membran gestaltet und sieht somit keine aktive Stellbeweglichkeit vor. Stattdessen wird die zweite Membran 24b zur Förderung der Dialysierflüssigkeit über das Koppelelement 26b und die inkompressible Koppelflüssigkeit 25b der mittels der aktiven Membranschicht ES induzierten Auslenkung der ersten Membran 23b nachgeführt.

Die beispielhafte Bilanzkammereinheit 7c nach Fig. 7 unterscheidet sich im Wesentlichen dadurch von den Bilanzkammereinheiten 7, 7a, 7b dass anstelle eines in die Membraneinheit 10c integrierten Aktorelements vielmehr ein abseits der Membraneinheit 10c angeordnetes Aktorelement in Gestalt eines Linearaktors Ec vorgesehen ist. Der Linearaktor ermöglicht eine Stellbewegung Rc, die bei der gezeigten Ausführungsform parallel zu einer Auslenkung der Membraneinheit 10c orientiert ist. Der Linearaktor Ec ist in grundsätzlicher Übereinstimmung mit dem Aktorelement E der Bilanzkammereinheiten 7, 7a, 7c aus einem dielektrischen Elastomermaterial M gebildet und insoweit ein dielektrischer Elastomeraktor. Allerdings ist keine planare, schichtförmig in die Membraneinheit 10c integrierte, sondern die anhand Fig. 7 schematisch verdeutlichte abseits angeordnete Konfiguration vorgesehen. Der Linearaktor Ec kann hierfür insbesondere als Bündelaktor gestaltet sein, der vergleichsweise hohe Stellkräfte bei großen Stellbewegungen ermöglicht.

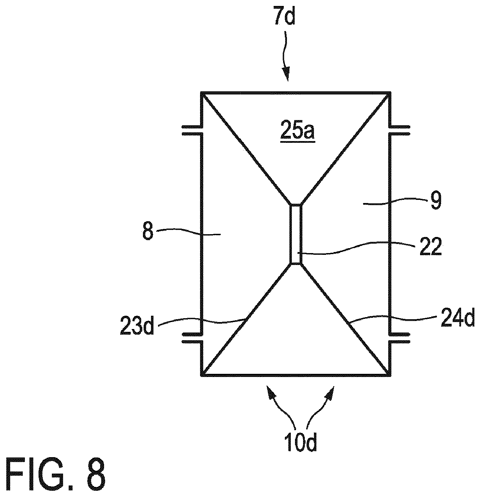

Die Bilanzkammereinheit 7d nach Fig. 8 ist weitestgehend identisch mit der Bilanzkammereinheit 7a nach Fig. 5 gestaltet. Im Unterschied zu deren Membraneinheit 10a weist die Membraneinheit 10d kein integriertes Federelement auf. Stattdessen ist sowohl die erste Membran 23d als auch die zweite Membran 24d der Membraneinheit 10d mit einer aktiven Membranschicht ES (Fig. 4) versehen.

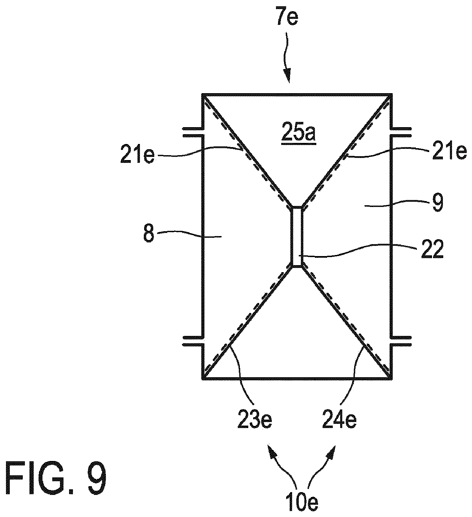

Die Bilanzkammereinheit 7e nach Fig. 9 ist ebenfalls weitestgehend identisch mit der Bilanzkammereinheit 7a nach Fig. 5. Im Unterschied zu der Membraneinheit 10a der Bilanzkammereinheit 7a weist die Bilanzkammereinheit 10e anstelle des extern angeordneten Federelements 21 zwei in die erste Membran 23e und die zweite Membran 24e integrierte Federelemente 21e auf. Die Federelemente 21e sind jeweils als Blattfeder gestaltet. Zur besseren Verdeutlichung sind die Federelemente 21e in Fig. 9 strichliert eingezeichnet.

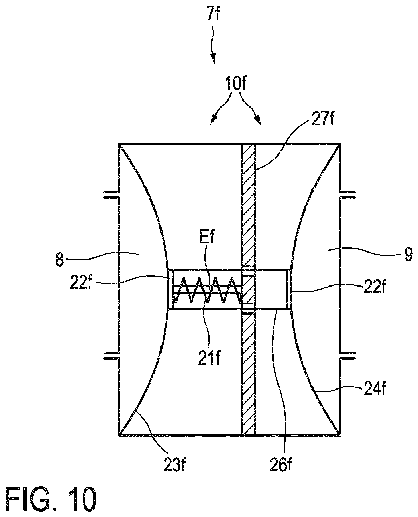

Die Bilanzkammereinheit 7f nach Fig. 10 weist eine Membraneinheit 10f mit einer ersten Membran 23f und einer zweiten Membran 24f auf, die mittels eines Koppelelements 26f zwecks Bewegungsübertragung miteinander gekoppelt sind. Das Koppelelement 26f greift an Ankerplatten 22f der ersten Membran 23f und der zweiten Membran 24f an. In Übereinstimmung mit der Ausführungsform nach Fig. 7 ist das Aktorelement in Gestalt eines Linearaktors Ef ausgebildet. Im Unterschied zu dem Linearaktor Ec der Bilanzkammereinheit 7c nach Fig. 7 ist der Linearaktor Ef in das nicht näher bezeichnete Gehäuse der Bilanzkammereinheit 7f integriert und einends an einer Stützplatte 27f und andernends an der Ankerplatte 22f der ersten Membran 23f abgestützt. Die Stützplatte 27f ist in einer dem Fachmann bekannten Weise innerhalb des Gehäuses festgelegt, so dass eine Kraftübertragung zwischen dem Gehäuse und dem Linearaktor Ef erreicht ist. Zudem ist ein Federelement 21f vorgesehen, das einends an der Stützplatte 27f und andernends an der Ankerplatte 22f abgestützt ist. Das Koppelelement 26f ist vorliegend rohrförmig gestaltet und in Axialrichtung zur Anbindung an die Ankerplatte 22f der zweiten Membran 24f durch einen nicht näher bezeichneten Durchlass der Stützplatte 27f erstreckt.

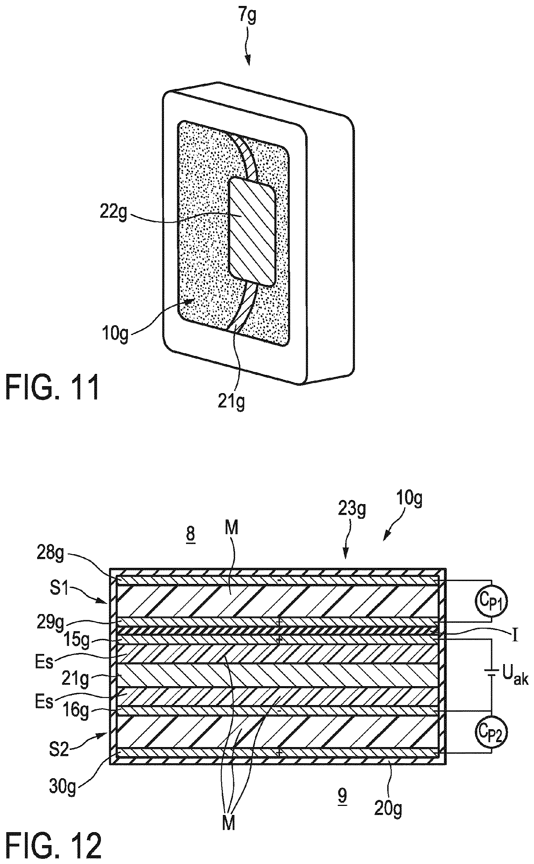

Anhand der Fig. 11 und 12 ist eine weitere Ausführungsform mit einer Bilanzkammereinheit 7g ersichtlich. Diese weist eine Membraneinheit 10g auf, deren Schichtaufbau in schematisch stark vereinfachter Weise anhand Fig. 12 gezeigt ist. Die Membraneinheit 10g gestattet zusätzlich zu der aktiven Stellbeweglichkeit eine Druckerfassung des in der ersten Bilanzkammer 8 und des in der zweiten Bilanzkammer 9 vorherrschenden Drucks.

Zur aktiven Stellbeweglichkeit der Membraneinheit 10g sind bei der gezeigten Ausführungsform zwei aktive Membranschichten ES in den Schichtaufbau integriert. Die aktiven Membranschichten ES sind durch ein Federelement 21g in Dickenrichtung der Membraneinheit 10g voneinander getrennt. Das Federelement 21g ist bei der gezeigten Ausführungsform in Form einer Blattfeder gestaltet. In Bezug auf die Zeichenebene der Fig. 12 sind die beiden Membranschichten ES ober- und unterseitig mit Elektroden 15g, 16g versehen. Die Elektroden 15g, 16g sind elektrisch leitfähig und in einer dem Fachmann bekannten Weise auf das Elastomermaterial M der aktiven Membranschichten ES aufgebracht.

In Dickenrichtung der Membraneinheit 10g oberhalb der aktiven Membranschichten ES ist eine erste Sensorschicht S1 in die Membraneinheit 10g integriert. Unterhalb der aktiven Membranschichten ES ist eine zweite Sensorschicht S2 in die Membraneinheit 10g integriert.

Die beiden Sensorschichten S1, S2 sind zur Erfassung des jeweiligen Drucks in der ersten Bilanzkammer 8 bzw. in der zweiten Bilanzkammer 9 eingerichtet. Die beiden Sensorschichten S1, S2 sind jeweils aus dem dielektrischen Elastomermaterial M gebildet. Der weitere Schichtaufbau der Membraneinheit 10g wird nachfolgend in Bezug auf die Zeichenebene der Fig. 12 erläutert, wobei die Oberseite der Membraneinheit 10g der ersten Bilanzkammer 8 zugewandt ist und die Unterseite der Membraneinheit 10g der zweiten Bilanzkammer 9 zugewandt ist.

Die erste Sensorschicht S1 ist an ihrer Oberseite mit einer oberen Elektrode 28g versehen. Unterseitig ist die erste Membranschicht S1 mit einer unteren Elektrode 29g versehen. Die untere Elektrode 29g ist mittels einer Isolationsschicht I elektrisch gegenüber der oberen Elektrode 15g der aktiven Membranschicht ES isoliert. Die zweite Sensorschicht S2 ist unterseitig mit einer unteren Elektrode 30g versehen. Die an der Unterseite der aktiven Membranschicht ES angeordnete untere Elektrode 16g fungiert gleichzeitig als Elektrode für die zweite Sensorschicht S2.

Eine weitere Membranschicht 20g bedeckt den vorbeschriebenen Membranaufbau umlaufend. Die weitere Membranschicht 20g ist vorliegend in Form einer Silikonummantelung ausgebildet.

Zur Ermittlung des Drucks in der ersten Bilanzkammer 8 und in der zweiten Bilanzkammer 9 wird der kapazitive Effekt der Sensorschichten S1, S2 ausgenutzt. Zu diesem Zweck wird eine konstante Spannung C_{P2} zwischen den Elektroden 16g und 30g der zweiten Sensorschicht S2 angelegt. Zudem wird eine konstante Spannung C_{P1} zwischen den Elektroden 28g und 29g der ersten Sensorschicht S1 angelegt.

Die Ladung des anhand Fig. 12 ersichtlichen Kondensators berechnet sich bekanntlich aus Q=CxU. Wird die untere Sensorschicht S2 durch einen in der zweiten Bilanzkammer 9 vorherrschenden Druck zusammengedrückt, ändert sich die Kapazität C und somit auch die Ladung Q. Aus der veränderten Ladung Q kann über I=dQ/dt eine Stromänderung gemessen werden, die proportional zum vorherrschenden Druck ist. Da die Bewegung der Membraneinheit 10g über eine kapazitive Erfassung der Auslenkungsposition mittels der aktiven Membranschichten ES bekannt ist, kann dies entsprechend berücksichtigt werden. Das heißt bei einer Signalverarbeitung zur Ermittlung des Drucks kann ein aus der Bewegung der Membraneinheit 10g herrührender Signalanteil quasi abgezogen werden, so dass lediglich ein dem Druck äquivalenter Signalanteil verbleibt.

Die beiden Sensorschichten S1, S2 müssen nicht zwingend über eine ganze Fläche der Membraneinheit 10g ausgebildet sein. Vielmehr ist es ausreichend, wenn die erste Sensorschicht S1 und/oder die zweite Sensorschicht S2 als Streifen, Kreuz oder dergleichen angeordnet sind. Je nach Breite des Streifens bzw. des Kreuzes ist eine umso größere kapazitive Fläche vorhanden, so dass die zuvor beschriebene Druckermittlung mit vergleichsweise höherer Genauigkeit erfolgen kann.

Es versteht sich, dass die einzelnen Merkmale der Bilanzkammereinheiten 7, 7a bis 7g im von den Ansprüchen vorgegebenen Rahmen miteinander kombinierbar sind. Beispielsweise kann bei der Bilanzkammereinheit 7c in erfindungsgemäßer Weise anstelle des zeichnerisch dargestellten Aufbaus der Membraneinheit 10c ein den Fig. 5, 6 entsprechender Aufbau mit einer ersten Membran und einer zweiten Membran sowie einer entsprechenden volumetrischen Kopplung vorgesehen sein. Im Übrigen ist die in den Fig. gezeigte Formgebung der Bilanzkammereinheiten als schematisch stark vereinfacht und rein exemplarisch aufzufassen. Alternative Formgebungen und/oder Geometrien, bspw. (vier-)eckig, oval, rund, sind vorstellbar.

Bei dem Bilanzkammersystem 6 nach Fig. 2 sind die erste Bilanzkammereinheit 7 und die zweite Bilanzkammereinheit 11 baugleich und jeweils gemäß der Ausführung nach den Fig. 3 und 4 gestaltet. Alternativ kann eine Gestaltung gemäß den Fig. 5 bis 12 vorgesehen sein.

Durch die vorbeschriebene Gestaltung der ersten Bilanzkammereinheit 7 - und vorliegend auch der zweiten Bilanzkammereinheit 11 - kann eine maßgebliche Vereinfachung des Bilanzkammersystems 6 gegenüber dem aus dem Stand der Technik bekannten Bilanzkammersystem 106 nach Fig. 1 erreicht werden. Denn anstelle der dort notwenigen magnetisch schaltbaren Ventile V1 bis V8 sieht das Bilanzkammersystem 6 einfache Rückschlagventile T1 bis T8 vor. Die Rückschlagventile T1 bis T8 können insbesondere als Tellerventile gestaltet sein. Die Rückschlagventile T1 bis T8 erfordern keine gesonderte Ansteuerung. Vielmehr sind die Rückschlagventile T1 bis T8 selbstbetätigt. D.h. die Rückschlagventile T1 bis T8 öffnen und schließen jeweils selbsttätig in Abhängigkeit der bei der Förderung der Dialysierflüssigkeit vorherrschenden Strömungsverhältnisse. Dabei sind die Rückschlagventile T1 bis T8 zu- und ablaufseitig der jeweiligen Bilanzkammer mit zueinander entgegengesetzten Öffnungs- bzw. Schließrichtungen installiert. Die jeweilige Öffnungsrichtung ist anhand der in Fig. 2 in unmittelbarer Nähe zu dem jeweiligen Rückschlagventil T1 bis T8 eingezeichneten Pfeile verdeutlicht. Es versteht sich, dass die erste Bilanzkammereinheit 7 und die zweite Bilanzkammereinheit 11 selbstverständlich auch bei einem Bilanzkammersystem mit magnetisch schaltbaren Schaltventilen verwendbar sind. Entsprechendes gilt für die in den Fig. 5 bis 12 gezeigten Ausgestaltungen. Insoweit sind die hier beschriebenen Rückschlagventile T1 bis T8 nicht zwingend notwendig, aber besonders vorteilhaft.

Die Funktion des Bilanzkammersystems 6 ist im Wesentlichen in zwei Phasen unterteilt, nämlich eine erste Phase und eine zweite Phase.

In der ersten Phase liegen die folgenden Startbedingungen vor: Erste Bilanzkammer 8 leer, zweite Bilanzkammer 9 voll, die Membraneinheit 10 nimmt eine nach links ausgelenkte Auslenkungsposition ein, dritte Bilanzkammer 12 voll, vierte Bilanzkammer 13 leer, die Membraneinheit 14 nimmt eine nach rechts ausgelenkte Auslenkungsposition ein, die Rückschlagventile T1 bis T8 sind geschlossen. Hiervon ausgehend werden die Membraneinheiten 10, 14 unter Ansteuerung ihres jeweiligen Aktorelements und dessen Stellbewegung aktiv - und nicht etwa von außen induziert wie bei dem Bilanzkammersystem 106 - ausgelenkt. Die Auslenkung erfolgt synchron entgegengesetzt unter kapazitiver Erfassung der Auslenkungsposition mittels des jeweiligen Aktorelements. Dementsprechend wird die Membraneinheit 10 der ersten Bilanzkammer 7 aktiv nach rechts ausgelenkt; die Membraneinheit 14 der zweiten Bilanzkammereinheit 11 wird aktiv und in einer auf die Auslenkung der Membraneinheit 10 abgestimmten Weise aktiv nach links ausgelenkt. Zur entsprechenden Ansteuerung der Aktorelemente kann eine hierfür eingerichtete Steuereinrichtung vorgesehen sein. Aufgrund der vorbeschriebenen Auslenkung der Membraneinheiten 10, 14 wird die erste Bilanzkammer 8 durch das - hierbei selbsttätig öffnende - Rückschlagventil T3 mit frischer Dialysierflüssigkeit befüllt. Dadurch wird verbrauchte Dialysierflüssigkeit, die sich in der zweiten Bilanzkammer 9 befindet, durch das - hierbei selbsttätig öffnende - Rückschlagventil T2 in den Abfluss A gefördert. Die Rückschlagventile T1 und T4 bleiben hierbei geschlossen, sodass ein nicht funktionsgerechtes Ausströmen von frischer Dialysierflüssigkeit aus der ersten Bilanzkammer 8 und von verbrauchter Dialysierflüssigkeit aus der zweiten Bilanzkammer 9 verhindert ist. Gleichzeitig wird aufgrund der synchron entgegengesetzten Auslenkung der Membraneinheit 11 verbrauchte Dialysierflüssigkeit durch das Rückschlagventil T6 in die vierte Bilanzkammer 13 gefördert und frische Dialysierflüssigkeit durch das Rückschlagventil T7 aus der dritten Bilanzkammer 12 in Richtung des Dialysators 2 gefördert. Die Rückschlagventile T5, T8 bleiben hierbei geschlossen. Hieran schließt sich die zweite Phase an. Diese beginnt bei Erreichen einer jeweiligen Endlage der Auslenkung der Membraneinheiten 10, 14 und insoweit bei folgenden Startbedingungen: Erste Bilanzkammer 8 voll, zweite Bilanzkammer 9 leer, dritte Bilanzkammer 12 leer, vierte Bilanzkammer 13 voll, wobei die Membraneinheit 10 nach rechts und die Membraneinheit 14 hierzu synchron entgegengesetzt nach links ausgelenkt ist. Ausgehend von diesen Startbedingungen erfolgt eine wiederrum synchrone und zueinander entgegensetzte Auslenkung der Membraneinheiten 10, 14 unter entsprechender Ansteuerung des jeweiligen Aktorelements.

Im Übrigen ist eine Ultrafiltrationspumpe P3 vorgesehen und in einer der Fig. 1 entsprechenden Weise angeordnet.

## Patentansprüche

1. Bilanzkammersystem (6) für ein Dialysegerät (1) mit einem Dialysator (2) zur extrakorporalen Blutbehandlung, aufweisend wenigstens eine zum fluidleitenden Anschluss an den Dialysator (2) vorgesehene Bilanzkammereinheit (7, 7a bis 7g), die eine erste Bilanzkammer (8), welche zur Aufnahme und Abgabe von frischstromseitiger Dialysierflüssigkeit vorgesehen ist, und eine zweite Bilanzkammer (9), welche zur Aufnahme und Abgabe von brauchstromseitiger Dialysierflüssigkeit vorgesehen ist, aufweist, wobei die erste Bilanzkammer (8) und die zweite Bilanzkammer (9) mittels einer zwischen unterschiedlichen Auslenkungspositionen auslenkbaren Membraneinheit (10, 10a bis 10g) fluiddicht voneinander getrennt und volumetrisch miteinander gekoppelt sind, wobei wenigstens ein aus einem dielektrischen Elastomermaterial (M) gebildetes Aktorelement (E, Ec, Ef) vorgesehen ist, das unter Einwirkung einer elektrischen Spannung eine Stellbewegung zur Auslenkung der Membraneinheit (10, 10a bis 10g) und damit zur Förderung der frischstrom- und brauchstromseitigen Dialysierflüssigkeit ausführt, **dadurch gekennzeichnet, dass** die Membraneinheit (10a, 10b, 10d, 10e, 10f) eine die erste Bilanzkammer (8) begrenzende erste Membran (23a, 23b, 23d, 23e, 23f) aufweist und eine die zweite Bilanzkammer (9) begrenzende zweite Membran (24a, 24b, 24d, 24e, 24f) aufweist, wobei die erste Membran (23a, 23b, 23d, 23e, 23f) und die zweite Membran (24a, 24b, 24d, 24e, 24f) zur volumetrischen Kopplung der ersten Bilanzkammer (8) mit der zweiten Bilanzkammer (9) mittels eines starren Koppelelements (26b, 26f) und/oder einer inkompressiblen Koppelflüssigkeit (25a, 25b) miteinander wirkverbunden sind.

2. Bilanzkammersystem (6) nach Anspruch 1, **dadurch gekennzeichnet, dass** das wenigstens eine Aktorelement (E, Ec, Ef) zur kapazitiven Erfassung der Auslenkungsposition der Membraneinheit (10, 10a bis 10g) eingerichtet ist und somit zusätzlich als Sensorelement fungiert.

3. Bilanzkammersystem (6) nach Anspruch 1 oder 2, **dadurch gekennzeichnet, dass** wenigstens ein Federelement (21, 21e, 21f, 21g) mit der Membraneinheit (10, 10a, 10b, 10c, 10e, 10f, 10g) und dem Aktorelement (E, Ec, Ef) wirkverbunden ist, wobei die Membraneinheit (10, 10a, 10b, 10c, 10e, 10f, 10g) - bei an dem Aktorelement (E, Ec, Ef) angelegter elektrischer Spannung - mittels des Federelements (21, 21e, 21f, 21g) in eine erste Richtung (X1) ausgelenkt ist, und wobei die Membraneinheit (10, 10a, 10b, 10c, 10e, 10f, 10g) - bei verringerter elektrischer Spannung - mittels des Aktorelements (E, Ec, Ef) entgegen der Einwirkung des Federelements (21, 21e, 21f, 21g) in eine der ersten Richtung (X1) entgegengesetzte zweite Richtung (X2) ausgelenkt ist.

4. Bilanzkammersystem (6) nach einem der vorhergehenden Ansprüche, **dadurch gekennzeichnet, dass** ein einziges Aktorelement (E, Ef) vorgesehen ist, dessen Stellbewegung mittels des Koppelelements (26b, 26f) und/oder der Koppelflüssigkeit (25b) zwischen der ersten Membran (23b, 23f) und der zweiten Membran (24b, 24f) übertragen ist.

5. Bilanzkammersystem (6) nach einem der vorhergehenden Ansprüche, **dadurch gekennzeichnet, dass** das wenigstens eine Aktorelement (E) in Gestalt wenigstens einer aktiven Membranschicht (ES) in die Membraneinheit (10, 10a, 10b, 10d, 10e, 10g), insbesondere in die erste Membran (23a, 23b, 23d, 23e, 23g) und/oder die zweite Membran (24a, 24b, 24d, 24e, 24g), integriert ist.

6. Bilanzkammersystem (6) nach Anspruch 5, **dadurch gekennzeichnet, dass** die aktive Membranschicht (ES) mittels wenigstes einer weiteren Membranschicht (19, 20, 20g) und/oder einer Beschichtung gegenüber einer Benetzung mit der Dialysierflüssigkeit geschützt ist.

7. Bilanzkammersystem (6) nach Anspruch 5 oder 6, **dadurch gekennzeichnet, dass** wenigstens eine aus einem dielektrischen Elastomermaterial (M) gebildete Sensorschicht (S1, S2) in die Membraneinheit (10g) integriert ist, wobei die wenigstens eine Sensorschicht (S1, S2) zur Erfassung eines in der ersten Bilanzkammer (8) und/oder der zweiten Bilanzkammer (9) vorherrschenden Drucks eingerichtet ist.

8. Bilanzkammersystem (6) nach einem der Ansprüche 1 bis 4, **dadurch gekennzeichnet, dass** das wenigstens eine Aktorelement in Gestalt eines Linearaktors (Ec, Ef) mit einer translatorischen Stellbewegung (Rc) abseits der Membraneinheit (10c, 10f) angeordnet ist.

9. Bilanzkammersystem (6) nach einem der vorhergehenden Ansprüche, **dadurch gekennzeichnet, dass** die Bilanzkammern (8, 9) jeweils einen Zulauf und einen Ablauf aufweisen, wobei zur Flusssteuerung durch den jeweiligen Zu- oder Ablauf jeweils ein Rückschlagventil (T1) bis (T8) vorgesehen ist.

10. Dialysegerät (1) mit einem Dialysator (2) zur extrakorporalen Blutbehandlung, wobei ein Bilanzkammersystem (6) nach einem der vorhergehenden Ansprüche fluidleitend an eine Dialysierflüssigkeitskammer (4) des Dialysators (2) angeschlossen ist.

## Claims

1. Balance chamber system (6) for a dialysis device (1) having a dialyser (2) for extracorporeal blood treatment, comprising at least one balance chamber unit (7, 7a to 7g) that is provided for fluid-conducting connection to the dialyser (2) and comprises a first balance chamber (8), which is provided for receiving and dispensing fresh-flow-side dialysis fluid, and a second balance chamber (9), which is provided for receiving and dispensing used-flow-side dialysis fluid, wherein the first balance chamber (8) and the second balance chamber (9) are fluid-tightly separated from one another and volumetrically coupled to one another by means of a membrane unit (10, 10a to 10g) that is deflectable between different deflection positions, wherein at least one actuator element (E, Ec, Ef) formed from a dielectric elastomer material (M) is provided and, under the influence of a voltage, performs an actuating movement for deflecting the membrane unit (10, 10a to 10g) and hence for conveying the fresh-flow-side dialysis fluid and the used-flow-side dialysis fluid, **characterized in that** the membrane unit (10a, 10b, 10d, 10e, 10f) comprises a first membrane (23a, 23b, 23d, 23e, 23f) that delimits the first balance chamber (8) and a second membrane (24a, 24b, 24d, 24e, 24f) that delimits the second balance chamber (9), wherein the first membrane (23a, 23b, 23d, 23e, 23f) and the second membrane (24a, 24b, 24d, 24e, 24f) are operatively connected to one another by means of a rigid coupling element (26b, 26f) and/or an incompressible coupling fluid (25a, 25b) for volumetric coupling of the first balance chamber (8) with the second balance chamber (9).

2. Balance chamber system (6) according to Claim 1, **characterized in that** the at least one actuator element (E, Ec, Ef) is configured for capacitive detection of the deflection position of the membrane unit (10, 10a to 10g) and thus additionally acts as sensor element.

3. Balance chamber system (6) according to Claim 1 or 2, **characterized in that** at least one spring element (21, 21e, 21f, 21g) is operatively connected to the membrane unit (10, 10a, 10b, 10c, 10e, 10f, 10g) and the actuator element (E, Ec, Ef), wherein the membrane unit (10, 10a, 10b, 10c, 10e, 10f, 10g) - when voltage is applied to the actuator element (E, Ec, Ef) - is deflected in a first direction (X1) by means of the spring element (21, 21e, 21f, 21g), and wherein the membrane unit (10, 10a, 10b, 10c, 10e, 10f, 10g) - in the event of reduced voltage - is deflected against the action of the spring element (21, 21e, 21f, 21g) in a second direction (X2), opposite to the first direction (X1), by means of the actuator element (E, Ec, Ef).

4. Balance chamber system (6) according to any of the preceding claims, **characterized in that** a single actuator element (E, Ef) is provided, the actuating movement of which is transmitted by means of the coupling element (26b, 26f) and/or the coupling fluid (25b) between the first membrane (23b, 23f) and the second membrane (24b, 24f).

5. Balance chamber system (6) according to any of the preceding claims, **characterized in that** the at least one actuator element (E) in the form of at least one active membrane layer (ES) is integrated into the membrane unit (10, 10a, 10b, 10d, 10e, 10g), in particular into the first membrane (23a, 23b, 23d, 23e, 23g) and/or the second membrane (24a, 24b, 24d, 24e, 24g).

6. Balance chamber system (6) according to Claim 5, **characterized in that** the active membrane layer (ES) is protected against wetting by the dialysis fluid by means of at least one further membrane layer (19, 20, 20g) and/or a coating.

7. Balance chamber system (6) according to Claim 5 or 6, **characterized in that** at least one sensor layer (S1, S2) formed from a dielectric elastomer material (M) is integrated into the membrane unit (10g), wherein the at least one sensor layer (S1, S2) is configured for detecting a prevailing pressure in the first balance chamber (8) and/or the second balance chamber (9).

8. Balance chamber system (6) according to any of Claims 1 to 4, **characterized in that** the at least one actuator element in the form of a linear actuator (Ec, Ef) with a translational actuating movement (Rc) is arranged away from the membrane unit (10c, 10f).

9. Balance chamber system (6) according to any of the preceding claims, **characterized in that** the balance chambers (8, 9) each have an inlet and an outlet, wherein a respective check valve (T1) to (T8) is provided for flow control through the respective inlet or outlet.

10. Dialysis device (1) having a dialyser (2) for extracorporeal blood treatment, wherein a balance chamber system (6) according to any of the preceding claims is fluid-conductively connected to a dialysis fluid chamber (4) of the dialyser (2).

## Revendications

1. Système (6) à chambres d'équilibrage pour une machine de dialyse (1) comprenant un dialyseur (2) pour le traitement extracorporel du sang, présentant au moins une unité de chambre d'équilibrage (7, 7a à 7g) prévue pour une liaison fluidique avec le dialyseur (2), qui comprend une première chambre d'équilibrage (8) prévue pour recevoir et délivrer du liquide de dialyse du côté du flux frais, et une deuxième chambre d'équilibrage (9) prévue pour recevoir et délivrer du liquide de dialyse du côté du flux utile, la première chambre d'équilibrage (8) et la deuxième chambre d'équilibrage (9) étant séparées l'une de l'autre de manière étanche aux fluides au moyen d'une unité à membranes (10, 10a à 10g) apte à être déviée entre différentes positions de déviation et étant reliées l'une à l'autre de manière volumétrique, au moins un élément actionneur (E, Ec, Ef) formé d'un matériau élastomère diélectrique (M) étant prévu, lequel, sous l'effet d'une tension électrique, effectue un mouvement d'actionnement pour dévier l'unité à membranes (10, 10a à 10g) et pour, de ce fait, transporter le liquide de dialyse côté flux frais et côté flux utile, **caractérisé en ce que** l'unité à membranes (10a, 10b, 10d, 10e, 10f) présente une première membrane (23a, 23b, 23d, 23e, 23f) délimitant la première chambre d'équilibrage (8) et une deuxième membrane (24a, 24b, 24d, 24e, 24f) délimitant la deuxième chambre d'équilibrage (9), la première membrane (23a, 23b, 23d, 23e, 23f) et la deuxième membrane (24a, 24b, 24d, 24e, 24f) étant reliées entre elles de manière à agir l'une sur l'autre pour une liaison volumétrique de la première chambre d'équilibrage (8) avec la deuxième chambre d'équilibrage (9) au moyen d'un élément de liaison rigide (26b, 26f) et/ou d'un liquide de liaison incompressible (25a, 25b).

2. Système (6) à chambres d'équilibrage selon la revendication 1, **caractérisé en ce que** ledit au moins un élément actionneur (E, Ec, Ef) est conçu de façon à détecter de manière capacitive la position de déviation de l'unité à membranes (10, 10a à 10g) et fonctionne ainsi également en tant qu'élément de détection.

3. Système (6) à chambres d'équilibrage selon la revendication 1 ou la revendication 2, **caractérisé en ce que** ledit au moins un élément ressort (21, 21e, 21f, 21g) est en liaison fonctionnelle avec l'unité à membranes (10, 10a, 10b, 10c, 10e, 10f, 10g) ainsi qu'avec l'élément actionneur (E, Ec, Ef), l'unité à membranes (10, 10a, 10b, 10c, 10e, 10f, 10g) - lorsque l'élément actionneur (E, Ec, Ef) est sous tension électrique - étant déviée dans une première direction (X1) au moyen de l'élément ressort (21, 21e, 21f, 21g), et l'unité à membranes (10, 10a, 10b, 10c, 10e, 10f, 10g) - lorsque la tension électrique est réduite - étant déviée au moyen de l'élément actionneur (E, Ec, Ef) à l'encontre de l'action de l'élément ressort (21, 21e, 21f, 21g) dans une deuxième direction (X2) opposée à la première direction (X1).

4. Système (6) à chambres d'équilibrage selon l'une des revendications précédentes, **caractérisé en ce qu'**un seul élément actionneur (E, Ef) est prévu, dont le mouvement d'actionnement est transmis entre la première membrane (23b, 23f) et la deuxième membrane (24b, 24f) au moyen de l'élément de liaison (26b, 26f) et/ou du liquide de liaison (25b).

5. Système (6) à chambres d'équilibrage selon l'une des revendications précédentes, **caractérisé en ce que** ledit au moins un élément actionneur (E) est intégré sous la forme d'au moins une couche de membrane active (ES) dans l'unité de membrane (10, 10a, 10b, 10d, 10e, 10g), en particulier dans la première membrane (23a, 23b, 23d, 23e, 23g) et/ou la deuxième membrane (24a, 24b, 24d, 24e, 24g).

6. Système (6) à chambres d'équilibrage selon la revendication 5, **caractérisé en ce que** la couche de membrane active (ES) est protégée contre le mouillage par le liquide de dialyse au moyen d'au moins une autre couche de membrane (19, 20, 20g) et/ou d'un revêtement.

7. Système (6) à chambres d'équilibrage selon la revendication 5 ou la revendication 6, **caractérisé en ce qu'**au moins une couche de détection (S1, S2) formée d'un matériau élastomère diélectrique (M) est intégrée dans l'unité à membranes (10g), ladite au moins une couche de détection (S1, S2) étant conçue de manière à détecter une pression régnant dans la première chambre d'équilibrage (8) et/ou la deuxième chambre d'équilibrage (9).

8. Système (6) à chambres d'équilibrage selon l'une des revendications 1 à 4, **caractérisé en ce que** ledit au moins un élément actionneur est agencé sous la forme d'un actionneur linéaire (Ec, Ef) ayant un mouvement d'actionnement translatoire (Rc) et étant disposé à l'écart de l'unité de membrane (10c, 10f).

9. Système (6) à chambres d'équilibrage selon l'une des revendications précédentes, **caractérisé en ce que** les chambres d'équilibrage (8, 9) comprennent chacune une entrée et une sortie, un clapet anti-retour (T1) à (T8) étant prévu de façon à réguler le débit à travers l'entrée ou la sortie respective.

10. Machine de dialyse (1) comprenant un dialyseur (2) pour le traitement extracorporel du sang, dans laquelle un système (6) à chambres d'équilibrage selon l'une des revendications précédentes est raccordé de manière fluidique à une chambre de liquide de dialyse (4) du dialyseur (2).
